# DEMANDE DE BREVET EUROPEEN

(11) **EP 2 018 893 A1**
(43) Date de publication de la demande: **28.01.2009**
(21) Numéro de dépôt: 08160600.6
(22) Date de dépôt: 17.07.2008
(51) Int. Cl.: A61Q 19/00, A61K 8/99, A61P 17/00, A61K 35/08, A61K 35/74

(54) **Utilisation d'au moins un extrait bactérien cultivé sur eau thermale pour le traitement des peaux, muqueuses et cuirs chevelus sensibles**

(30) Priorité: 17.07.2007 FR 0756551
(71) Demandeur: L'Oréal, 75008 Paris (FR)
(72) Inventeur: Breton, Lionel, 78000, VERSAILLES (FR); Mahe, Yann, 91700, SAINTE GENEVIEVE DES BOIS (FR); Martin, Richard, 37210, ROCHECORBON (FR)
(74) Mandataire: Allab, Myriam

(57) **Abrégé**

La présente invention se rapporte à l'utilisation d'au moins un extrait de bactérie filamenteuse non photosynthétique et non fructifiante cultivée sur eau thermale et/ou minérale non sulfureuse pour le traitement des peaux, muqueuses et cuirs chevelus sensibles.

## Description

La présente invention concerne l'utilisation d'extraits bactériens cultivés sur eau thermale pour le soin des peaux et/ou cuirs chevelus sensibles.

La peau humaine est constituée de deux compartiments, à savoir un compartiment profond, le derme et un compartiment superficiel, l'épiderme.

L'épiderme est en contact avec l'environnement extérieur. Un de ses rôles consiste à protéger l'organisme de la déshydratation et des agressions extérieures, notamment liées à des facteurs environnementaux de type agents irritants ou polluants (détergents, pollution, fumée de cigarettes...), sollicitations mécaniques (frottements, abrasion, rasage, lavage fréquent...), déséquilibres thermiques ou climatiques (froid, vent, sécheresse, radiations UV...), xénobiotiques (micro-organismes, allergènes...), traitements chimiques cosmétiques ou dermatologiques (peeling, traitement anti-acné...), ou par des facteurs physiologiques (âge, stress...).

Certains individus présentent une peau ou un cuir chevelu plus sensible que d'autres. D'une manière générale, les peaux sensibles se définissent par une réactivité particulière de la peau. Toutefois, par opposition aux peaux qualifiées d'allergiques, cette réactivité ne relève pas d'un processus immunologique, c'est-à-dire ne se produit pas uniquement sur une peau déjà sensibilisée en réponse à la présence d'un allergène. Son mécanisme est dit aspécifique.
Cette réactivité cutanée se traduit classiquement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultraviolets ou infrarouges. Il existe également des facteurs associés comme l'âge et le type de peau. Ainsi les peaux sensibles sont plus fréquentes parmi les peaux sèches ou grasses que parmi les peaux normales.

L'apparition de ces signes d'inconfort, qui apparaissent dans les minutes qui suivent la mise en contact du sujet avec l'élément déclenchant, est une des caractéristiques essentielles des peaux sensibles. Il s'agit principalement de sensations dysesthésiques. On entend par sensations dysesthésiques, des sensations plus ou moins douloureuses ressenties dans une zone cutanée comme les picotements, fourmillements, démangeaisons ou prurits, , échauffements, inconforts, tiraillements, etc. Ces signes subjectifs existent le plus souvent en l'absence de signes chimiques visibles tels que la rougeur et les desquamations. On sait aujourd'hui que ces réactions d'irritation et d'intolérance cutanée sont notamment liées à une libération de neuropeptides par les terminaisons nerveuses de l'épiderme et du derme.
Les manifestations de la peau ou du cuir chevelu dits "sensibles" au sens de la présente invention apparaissent en dehors de toute réaction d'inflammation. On sait que l'inflammation se caractérise par 4 signes cliniques concomitants (rubor, calo, dolor, tumor) qui ne sont pas présents dans les phénomènes de peau ou de cuir chevelu sensible; en particulier, on n'observe pas de gonflement (tumor) dans les signes de peau sensible, et les sensations dysesthésiques, bien que correspondant à un inconfort pour le sujet qui les ressent, ne peuvent être qualifiées de douloureuses.

Pour des raisons évidentes, l'absence de signes visibles rend difficile le diagnostic de peau sensible. Le plus souvent ce diagnostic repose sur l'interrogatoire du patient. Cette symptomatologie a en outre pour intérêt de permettre de différencier la peau sensible associée ou non à une peau sèche, de l'irritation ou de l'allergie de contact pour lesquelles il existe en revanche des signes inflammatoires visibles.
En conséquence, la mise au point de produits "peaux sensibles" a nécessité de disposer d'outils d'évaluation de la réaction sensorielle de la peau. Les premiers outils se sont inspirés dès leur conception de la caractéristique essentielle des peaux sensibles à savoir présence de signes d'inconfort induits par une application topique. Ainsi, le "stinging test" à l'acide lactique a été le premier test proposé. Il est réalisé par relevé des sensations de picotements rapportées par un volontaire après application d'une solution d'acide lactique à 10 % sur les ailes du nez. Les sujets rapportant des sensations modérées ou fortes de picotements sont appelés "stingers" et considérés comme étant à peau sensible. En raison de cette sensibilité cutanée à l'application topique de produit, ces sujets sont alors sélectionnés pour tester des produits dits peaux sensibles. Plus récemment, pour activer spécifiquement les terminaisons nerveuses périphériques, impliquées dans l'inconfort et appelées nocicepteurs, récemment identifiées comme étant impliquées dans la peau sensible, de nouveaux tests ont été proposés qui utilisent précisément d'autres inducteurs d'inconfort comme la capsaïcine.
Ce second type de test, décrit dans la demande EP 1 374 913, constitue également un autre outil particulièrement utile pour le diagnostic de peaux sensibles.

Au sens de la présente invention, les peaux sensibles couvrent les peaux irritables et les peaux intolérantes.
Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.
Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température ou la laine.
Le plus souvent, l'irritabilité de la peau se traduit par des signes visibles tels qu'une rougeur cutanée, un sentiment d'échauffement de la peau ou du cuir chevelu (chaleur) pouvant aller jusqu'à un sentiment de douleur.

Les cuirs chevelus "sensibles" ont une sémiologie clinique plus univoque : les sensations de démangeaisons et/ou de picotements et/ou d'échauffements sont essentiellement déclenchées par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Il subsiste toujours le besoin de trouver de nouvelles compositions permettant de prévenir et/ou de traiter les symptômes des muqueuses, cuirs chevelus et peaux sensibles.

La demanderesse a mis en évidence une activité antagoniste de substance P d'un extrait bactérien cultivé sur eau thermale supérieure par rapport à l'extrait bactérien cultivé classiquement.

Ainsi l'invention se rapporte en premier lieu à l'utilisation cosmétique d'au moins un extrait de bactérie filamenteuse non photosynthétique et non fructifiante cultivée sur une eau minérale et/ou thermale non sulfureuse comme agent pour prévenir et/ou traiter les peaux sensibles.

### Extrait bactérien

L'extrait bactérien utilisé selon l'invention est préparé suivant un procédé comprenant la culture d'au moins une bactérie filamenteuse non photosynthétique et non fructifiante sur un milieu comprenant au moins une eau minérale et/ou thermale non sulfureuse.

Les bactéries utilisées sont des bactéries filamenteuses non photosynthétiques qui comprennent notamment les bactéries appartenant à l'ordre des Beggiatoales, et plus particulièrement les bactéries appartenant aux genres Beggiatoa, Vitreoscilla, Flexithrix ou Leucothrix.
Pour la mise en oeuvre de l'invention, on préfère les bactéries appartenant au genre Vitreoscilla, en particulier pour les bactéries de l'espèce *Vitreoscilla filiformis.*

Ces bactéries dont plusieurs ont déjà été décrites ont généralement un habitat aquatique, et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)
De préférence, la bactérie est celle correspondant à la souche déposée à l'ATCC sous le n° 15551.

Par eau thermale, on entend une eau chaude ou froide qui est utilisée pour ses vertus thérapeutiques ou pour un usage balnéaire. On peut utiliser une eau thermale ou une eau minérale. En général, une eau minérale est propre à la consommation, ce qui n'est pas toujours le cas d'une eau thermale. Chacune de ces eaux contient, entre autres, des minéraux solubilisés et des oligoéléments. Ces eaux sont connues pour être employées à des fins de traitement spécifique selon les oligo-éléments et les minéraux particuliers qu'elles contiennent.

De préférence, on utilise une eau thermale et/ou minérale qui présente une minéralisation supérieure ou égale à 400 mg/l.

On entend, dans l'invention, par "minéralisation", la somme des concentrations en anions et en cations présents dans l'eau thermale ou minérale. Dans les eaux thermales ou minérales utiles selon l'invention, la minéralisation est généralement comprise entre 400 et 900 mg/l.

L'eau thermale et/ou minérale utilisée selon l'invention peut avoir une minéralisation d'au moins 700 mg/l et, en particulier, une concentration totale en carbonates et en bicarbonates d'au moins 150 mg/l et plus préférentiellement d'au moins 360 mg/l et notamment en carbonate et bicarbonate de sodium supérieure à 2 mg/l. La concentration en oxyde de silicium dans l'eau utilisée dans la composition selon l'invention peut être de préférence d'au moins 6 mg/l et plus préférentiellement d'au moins 9 mg/l.

L'eau thermale ou l'eau minérale utilisée selon l'invention peut être choisie parmi l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, les Eaux Bonnes, l'eau de Rochefort, l'eau de Saint Christau, l'eau des Fumades et l'eau de Tercis-les-bains.

Avantageusement, elle est choisie parmi les eaux non sulfureuses telles que l'eau de Vittel, les eaux du bassin de Vichy, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, l'eau de Rochefort, l'eau de Saint Christau et l'eau de Tercis-les-bains.

Parmi ces eaux, celles qui présentent une concentration totale en carbonates ou bicarbonates supérieure à 360 mg/l sont l'eau de Vittel, l'eau de la Bourboule, l'eau des Fumades, l'eau d'Enghien-les-bains, l'eau de la Roche-Posay, l'eau du bassin de Vichy, l'eau d'Uriage.

Parmi ces eaux celles qui présentent une concentration en carbonates ou bicarbonates comprise entre 150 mg/l et 360 mg/l sont l'eau de Digne, l'eau de Maizières, l'eau de Rochefort, l'eau de Saint-Gervais-les-bains.

Parmi ces eaux, celles qui contiennent au moins 2 mg/l de carbonate ou bicarbonate de sodium sont l'eau de la Roche Posay, l'eau de Vittel, les eaux du bassin de Vichy, l'eau d'Uriage.

Les eaux contenant au moins 9 mg/l d'oxyde de silicium sont l'eau de la Roche Posay, l'eau de Vittel, les eaux du bassin de Vichy, l'eau d'Uriage.

Les eaux thermales ou minérales convenant particulièrement à la mise en oeuvre de l'invention ont une concentration en ions calcium supérieure ou égale à 100 mg/l, voire à 140 mg/l.
Selon un mode de réalisation avantageux, l'eau thermale ou minérale a une concentration en ions hydrogénocarbonates supérieure ou égale à 300 mg/l. Les hydrogénocarbonates, aussi appelés bicarbonates, sont notamment présents à une concentration supérieure ou égale à 350 mg/l.

Selon un mode de réalisation avantageux, les bactéries sont cultivées sur un milieu comprenant au moins une eau thermale. Celle-ci peut en particulier être choisie parmi l'eau de Vittel, les eaux du bassin de Vichy, l'eau d'Uriage, l'eau de la Roche-Posay, l'eau de la Bourboule, l'eau des Fumades, l'eau d'Enghien-les-bains, les Eaux Bonnes, et notamment parmi l'eau de Vittel, les eaux du bassin de Vichy, l'eau de la Roche-Posay, l'eau de la Bourboule et l'eau d'Enghien-les-bains.

Les eaux permettant d'obtenir un résultat particulièrement avantageux selon l'invention sont notamment choisies parmi l'eau de la Roche Posay et l'eau de Vittel, ou une eau de composition similaire.
L'eau de la Roche Posay est extraite de la source du même nom, il s'agit d'une eau bicarbonatée calcique, silicatée et séléniée. Elle comprend généralement environ 387 mg/l d'ions bicarbonates, environ 140 mg/l d'ions calcium et au moins 4 mg/l de sulfates.
L'eau de Vittel est riche en calcium et sels minéraux (841 mg/l) et contient notamment 202 mg/l de calcium, 402 mg/l de bicarbonates et 336 mg/l de sulfates.

On peut en particulier effectuer une culture dans le milieu suivant :

| **Composition** | **Concentration** |
|---|---|
| Extrait autolytique de levure | 0,5 à 5 g/l |
| Peptone végétale | 0,5 à 5 g/l |
| Glucose anhydre | 0,5 à 7 g/l |
| Micro-éléments de Heller | 0,5 à 5 ml/l |
| CaCl₂, 10 H₂O | 0,010 à 0,200 g/l |

On complète à 1000 ml par de l'eau minérale et/ou thermale éventuellement complétée d'eau distillée ou osmosée.

Parmi les peptones utilisables, on peut citer par exemple la peptone papaïnique de soja. Ce milieu se distingue des milieux généralement utilisés par l'absence de catalase et de sulfure.
Les micro-éléments de Heller ont été décrits par Heller, Ann Sci. Nat. Biol. Veg. 14 : 1-223 (1953). Il s'agit de mélanges de divers éléments minéraux qui ont été recommandés par Heller, non pas pour la culture des bactéries, mais pour la nutrition de tissus végétaux cultivés *in vitro.*

La culture peut être effectuée à la température appropriée convenant pour l'espèce bactérienne cultivée. Généralement cette température est comprise entre 18 et 40°C suivant les souches. Le pH du milieu de culture est de préférence compris entre 5,5 et 8.

La composition des micro-éléments de Heller, pour 1I d'eau, est la suivante:

| | |
|---|---|
| ZnSO₄,7 H₂O | 1 g |
| MnSO₄,H₂O | 0,076 g |
| CuSO₄,5H₂O | 0,003 g |
| Kl | 0,010 g |
| H₃BO₃ | 1 g |
| AlCl₃,6H₂O | 0,050 g |
| NiCl₂,6H₂O | 0,030 g |

Lesdites eaux thermales ou minérales peuvent représenter tout ou partie de la phase aqueuse du milieu de culture. Elles peuvent ainsi se trouver en mélange en toute proportion avec l'eau, en particulier distillée ou osmosée, présente dans le milieu de culture. Le mélange (i) d'eau thermale et (ii) d'eau osmosée ou distillée pourra être dans un ratio compris entre 0,1% et 100%, notamment de 0,1 à 50, en particulier de 0,1 à 25.

En particulier, un extrait de bactérie filamenteuse convenant à l'utilisation selon l'invention est susceptible d'être obtenu par culture de ladite bactérie sur un milieu de culture qui contient un mélange (i) d'eau osmosée ou distillée et (ii) d'eau thermale, dans un ratio (i)/(ii) compris entre 1 et 100, notamment de 1 à 50, en particulier de 1 à 25.
On utilisera en particulier l'eau thermale de La Roche Posay, telle que définie dans ce qui précède.
Après mélange de tous les éléments du milieu, on procède avantageusement à une stérilisation du milieu de culture contenant l'eau thermale et/ou minérale; cette étape est effectuée par des méthodes connues de l'homme du métier telles que la stérilisation par filtration ou par la chaleur.

Le milieu de culture est ensuite ensemencé par les bactéries.

Les milieux convenant le mieux à la culture des bactéries sont tels que l'eau thermale ou minérale représente de préférence au moins 0,1% de la quantité d'eau introduite pour la préparation du milieu, notamment de 0,1 à 99,9%. De bons résultats sont obtenus avec des concentrations d'eau thermale d'environ 1 ou 2%, notamment d'environ 1,33% par rapport à l'eau osmosée et/ou distillée, par exemple de 0,5 à 20%, voire de 0,5 à 50%, mais ces concentrations peuvent être augmentées sans inconvénient.

De façon connue, le procédé de préparation de l'extrait bactérien comprend au moins une étape dans laquelle on récupère les bactéries à la fin de la culture, en particulier en les séparant du milieu de culture.

Après culture des bactéries, on peut isoler la biomasse par diverses méthodes connues, par exemple par filtration, par coagulation avec un alcool (éthanol, isopropanol, isobutanol), par séchage sur cylindre à précouche (amidon, diatomées...) raclée, ou par lyophilisation. Une concentration préalable, par exemple à 80°C sous pression réduite, améliore cette séparation.
On peut procéder à une opération de rupture des enveloppes, par exemple par l'action des ultrasons. On peut en outre préparer des extraits à l'aide d'un alcool tel que l'éthanol ou le propanol.
La biomasse peut être utilisée vivante ou bien être traitée par différents procédés. On peut également préparer des extraits lipopolysaccharidiques selon les méthodes connues, voir par exemple Noris et Ribbons, Methods in Microbiology, Vol. 5B, Academic Press (1971). La méthode généralement utilisée est la méthode bien connue dite de Westphal (ou une méthode apparentée), qui consiste à faire l'extraction avec des mélanges phénol-eau à 65°C. On soumet ensuite l'extrait à une dialyse pour éliminer le phénol.

L'extrait bactérien utile selon l'invention peut encore résulter de la mise en oeuvre du procédé suivant : (i) on cultive au moins une bactérie appartenant à l'ordre des Beggiatoales sur un milieu comprenant un ose comme source principale de carbone et au moins une eau minérale ou thermale, puis (ii) après fermentation, on sépare les bactéries du milieu de culture, pour récupérer ladite masse des bactéries.

Les bactéries récupérées à l'issue de l'étape de fermentation peuvent notamment être soumises à un traitement de stabilisation et/ou d'extraction. C'est l'extrait de bactéries filamenteuses ainsi obtenu qui sera généralement utilisé dans ou pour la préparation de compositions cosmétiques ou dermatologiques. De manière connue en soi, l'extrait pourra ainsi être stérilisé en particulier par filtration ou par autoclavage.

Par extrait de bactéries filamenteuses non photosynthétiques, on entend aussi bien le surnageant de culture des dites bactéries, la biomasse obtenue après culture des dites bactéries ou encore les extraits de la biomasse obtenus par traitement de cette biomasse.
Pour préparer l'extrait selon l'invention, on peut cultiver lesdites bactéries selon le procédé selon l'invention, puis les séparer de la biomasse obtenue, par exemple par filtration, centrifugation, coagulation et/ou lyophilisation.

Ainsi, après culture, les bactéries peuvent être concentrées par centrifugation. La biomasse obtenue est autoclavée. Cette biomasse peut être lyophilisée pour constituer ce que l'on appelle l'extrait lyophilisé. Toute méthode de lyophilisation connue de l'homme du métier est utilisable pour préparer cet extrait.
La fraction surnageante de cette biomasse peut également être filtrée dans un récipient stérile pour éliminer les particules en suspension. Cette fraction surnageante peut également être transvasée stérilement dans un récipient stérile. Selon un mode de réalisation particulier de l'invention, la fraction surnageante ainsi obtenue est utilisée à titre d'actif cosmétique ou dermatologique.

L'extrait bactérien utile selon l'invention peut être formulé dans un support approprié à raison d'au moins 20% en poids par rapport au poids total de la composition, en particulier à raison de 0,001 à 20% en poids par rapport au poids total de la composition et plus particulièrement à raison de 0,01 à 10% en poids par rapport au poids total de la composition.
Pour certaines applications ou des formulations spécifiques, il peut être avantageux d'utiliser des concentrations pondérales élevées en extrait bactérien, par exemple, allant jusqu'à 15%, voire 20%.

L'extrait bactérien selon l'invention peut également être inclus sous forme de fractions de composants cellulaires ou sous la forme de métabolites. Le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Pour certaines applications, la biomasse vivante peut être utilisée telle qu'elle par exemple sous forme de masques ou de cataplasme pour produire un effet immédiat.

Au sens de l'invention, le terme "métabolite" désigne toute substance issue du métabolisme des microorganismes considérés selon l'invention et dotée d'une efficacité pour le traitement des peaux sensibles.

De manière inattendue, la Demanderesse a constaté que les extraits bactériens cultivés sur eau thermale pouvaient s'avérer efficaces pour le traitement des peaux, muqueuses, semi-muqueuses et cuirs chevelus sensibles ainsi que pour le traitement de signes associés aux peaux sensibles.

En effet, la Demanderesse a pu mettre en évidence que l'extrait de la bactérie *Vitreoscilla filiformis* cultivée sur l'eau thermale de la Roche Posay a une efficacité de traitement des signes des peaux sensibles accrue par rapport à l'extrait de la même bactérie cultivée sur milieu classique, c'est-à-dire sans eau minérale ou thermale.
La différence essentielle entre ces deux extraits réside dans les protocoles de préparation du milieu de culture où il y a substitution, au moins en partie, de l'eau osmosée par l'eau de La Roche Posay. Selon une hypothèse à laquelle n'est pas tenue la Demanderesse, cela conduirait notamment à une modification du métabolisme des bactéries causée par un enrichissement du milieu de culture en éléments minéraux, particulièrement en sélénium, strontium et zinc.

Il est également intéressant de noter que l'introduction de cette biomasse dans un support formulatoire n'entraîne pas de risque de surexposition à ces éléments, Se et Zn sont des éléments essentiels à l'organisme et le Sr est largement répandu dans l'alimentation.
Le tableau ci-dessous fournit les concentrations de ces éléments chimiques dans l'extrait bactérien selon l'invention préparé selon le protocole de l'exemple 1 (extrait lyophilisé).

| | |
|---|---|
| Se (mg/kg) | 6 |
| Sr (mg/kg) | 10 |
| Zn (mg/kg) | 216 |

Ainsi, l'application de cet extrait enrichi entraîne des expositions topiques en sels minéraux par jour de l'ordre de :

| | |
|---|---|
| Se (µg/J) | 0,008 |
| Sr (µg/J) | 0,0032 |
| Zn (µg/J) | 0,094 |

Rappelons ici que l'utilisation des ions pour l'amélioration de l'état cutané est très ancienne. Ainsi, les cures thermales à visée dermatologique sur les bords de la Mer Morte -étendue d'eau la plus saline du monde- remontent à l'Antiquité (Abels DJ et coll, Clinics in Dermatol 14 : 653-658,1996). Ces bains exercent une activité anti-prurigineuse et il n'est pas rare que les personnes traitées éprouvent le sentiment d'avoir une peau plus lisse et souple (Even-Pazz Z, Isr J Med Sci 32 :11-15, 1996). A ce jour, l'intérêt de l'application topique de cations a été autant étudié dans le domaine de la sensibilité que dans celui de la sécheresse cutanée. Parmi les cations divalents, c'est l'effet apaisant du strontium qui a été le plus documenté (Hahn GS, In biochemical modulation of skin reactions. Kydonieus AF, Will JJ (eds), CRC, Boca Raton, Florida, US, 261-272, 2000).

Cependant, l'enrichissement de la culture bactérienne en ions ne suffit pas à expliquer les meilleures propriétés de l'extrait selon l'invention, en effet, les essais comparatifs réalisés montrent une meilleure activité de l'extrait bactérien selon l'invention par rapport au même extrait bactérien cultivé classiquement auquel on ajoute des ions.

Plus spécifiquement, la présente invention se rapporte à l'utilisation cosmétique d'au moins un extrait de bactérie non photosynthétique et non fructifiante cultivée sur une eau minérale et/ou thermale non sulfureuse comme agent pour traiter les peaux et cuirs chevelus sensibles, notamment, les peaux intolérantes ou irritables.
Ainsi, l'extrait selon l'invention présente un intérêt comme agent apaisant.

Par traiter, sauf indication contraire, on entend toute action visant à améliorer le confort, le bien-être d'un individu, ce terme couvre donc aussi bien prévenir, atténuer, soulager que curer.

En particulier, l'invention se rapporte à l'utilisation d'au moins un extrait de bactérie non photosynthétique et non fructifiante cultivée sur une eau minérale et/ou thermale non sulfureuse comme agent pour traiter les sensations dysesthésiques des peaux sensibles, en particulier, les sensations choisies parmi les picotements et/ou les fourmillements et/ou les démangeaisons et/ou les échauffements et/ou l'inconfort cutané et/ou les tiraillements de la peau,

Un autre objet de la présente invention se rapporte à l'utilisation d'une quantité efficace d'au moins un extrait de bactérie non photosynthétique et non fructifiante cultivée sur une eau minérale et/ou thermale non sulfureuse pour la préparation d'une composition destinée à traiter les désordres associés à un excès de synthèse et/ou de libération de substance P, en particulier, les désordres cutanés.
L'invention se rapporte encore à moins un extrait de bactérie non photosynthétique et non fructifiante cultivée sur une eau minérale et/ou thermale non sulfureuse pour son utilisation dans le traitement des désordres associés à un excès de synthèse et/ou de libération de substance P, en particulier, les désordres cutanés.

Les compositions selon l'invention sont généralement administrées par voie topique ou par voie orale, de préférence par voie orale.
Les compositions selon l'invention peuvent se présenter sous toutes les formes galéniques normalement utilisées selon la voie d'utilisation.

Pour une application par voie topique, on entend une application sur la peau au sens large, c'est-à-dire sur toute surface cutanée du corps qu'elle soit ou non recouverte de poils ou de cheveu, notamment muqueuse, semi-muqueuse, cuir chevelu.

Une telle application par voie topique permet un traitement topique des peaux sensibles, incluant les lèvres et les cuirs chevelus sensibles, et des signes qui peuvent leur être associés.

En ce qui concerne les produits destinés à être appliqués topiquement en vue de produire l'effet recherché dans le cadre de l'invention, il peut s'agir de solutions aqueuses, hydroalcooliques ou huileuses, de dispersions du type des solutions ou dispersions du type lotion ou sérum, d'émulsions de consistance liquide ou semi-liquide du type lait, de suspensions ou émulsions, du type crème, de gel aqueux ou anhydre, de microémulsions, de microcapsules, de microparticules, ou de dispersions vésiculaires de type ionique et/ou non ionique.
Les compositions cosmétiques et/ou dermatologiques, plus particulièrement concernées par une application topique, peuvent se présenter notamment sous forme de solutions aqueuses, hydroalcooliques ou huileuses, de dispersions du type des solutions ou dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème, de gel aqueux ou anhydre, ou encore de microémulsions, de microcapsules, de microparticules, ou de dispersions vésiculaires de type ionique et/ou non ionique.
Ces compositions sont préparées selon les méthodes usuelles.
Ces compositions peuvent notamment constituer des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, ou des compositions contre les piqûres d'insectes.
Les compositions selon l'invention peuvent également consister en des préparations solides constituant des savons ou des pains de nettoyage.
Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.
Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique et/ou dermatologique. L'émulsionnant et le co-émulsionnant peuvent être présents, dans la composition, en une proportion allant de 0,3 à 30 % en poids, et de préférence de 0,5 à 20 % en poids par rapport au poids total de la composition.
Lorsque la composition de l'invention est une solution ou un gel huileux, la phase grasse peut représenter plus de 90 % du poids total de la composition.
De façon connue, la composition cosmétique et/ou dermatologique de l'invention peut contenir également des adjuvants habituels dans le domaine cosmétique pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Bien entendu, les compositions selon l'invention peuvent contenir plusieurs autres actifs. A titre d'actifs utilisables en association avec l'extrait bactérien selon l'invention, on peut citer, les vitamines B3, B5, B6, B8, C, E, ou PP et la niacine.
Comme actifs hydrophiles, on peut utiliser les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe vera.
Comme actifs lipophiles, on peut utiliser le rétinol (vitamine A) et ses dérivés, le tocophérol (vitamine E) et ses dérivés, les céramides, les huiles essentielles.
On peut, en outre, associer les actifs selon l'invention, à des agents actifs destinés notamment à la prévention et/ou au traitement des affections cutanées.

Selon un autre de ses objet, l'invention se rapporte à une composition comprenant au moins un extrait de bactérie non photosynthétique et non fructifiante cultivée sur une eau minérale et/ou thermale non sulfureuse en association avec au moins un autre inhibiteurs de neuropeptides, notamment un antagoniste de neuropeptide Y, un autre antagoniste de substance P, un antagoniste de GCRP, ou encore un inhibiteurs de NO-synthase et/ou un ingrédient susceptible de provoquer une irritation.

Des exemples d'inhibiteurs de NO-synthase convenant à une utilisation dans la présente invention comprennent notamment un extrait de végétal de l'espèce *Vitis vinifera* qui est notamment commercialisé par Euromed sous la dénomination Leucocyanidines de raisins extra, ou encore par Indena sous la dénomination Leucoselect^{®}, ou enfin par Hansen sous la dénomination Extrait de marc de raisin ; un extrait de végétal de l'espèce *Olea europaea* qui est de préférence obtenu à partir de feuilles d'olivier et est notamment commercialisé par VINYALS sous forme d'extrait sec, ou par Biologia & Technologia sous la dénomination commerciale Eurol BT ; et un extrait d'un végétal de l'espèce *Gingko biloba* qui est de préférence un extrait aqueux sec de ce végétal vendu par Beaufour sous le nom commercial Ginkgo biloba extrait standard.
La composition selon l'invention comprenant un inhibiteur de NO-synthase tel que défini ci-dessus peut avantageusement être utilisée pour prévenir ou traiter les signes du vieillissement cutané et/ou les peaux sensibles.

Parmi les composés susceptibles de provoquer une irritation cutanée, on peut notamment citer des composés ou actifs cosmétiques, des composés ou actifs dermatologiques, des tensioactifs notamment des tensioactifs anioniques, des conservateurs, des détergents, des parfums et notamment des solutions alcooliques parfumantes, des solvants, des propulseurs et leurs mélanges.
Plus particulièrement à titre d'actifs dermatologiques ou cosmétiques on peut citer certains agents desquamants qui peuvent être également des agents de peeling.
Parmi les agents spécifiquement de peeling on peut citer des particules abrasives/exfoliantes de sources minérales, organiques, naturelles ou synthétiques. On peut plus particulièrement citer les particules de pierre ponce, de silice, des billes de polyéthylènes, de nylon et des poudres de noyaux de fruits.
Parmi ces agents desquamants les suivants sont susceptibles de provoquer une irritation de la peau : les acides monocarboxyliques saturés (acide acétique) et insaturés, les acides dicarboxyliques saturés et insaturés, les acides tricarboxyliques saturés et insaturés ; les α-hydroxyacides et β-hydroxyacides des acides monocarboxyliques ; les α-hydroxyacides et β-hydroxyacides des acides dicarboxyliques ; les α-hydroxyacides et β-hydroxyacides, des acides tricarboxiliques, les cétoacides, les α-cétoacides, les β-cétoacides d'acides polycarboxyliques, d'acides polyhydroxy monocarboxyliques, d'acides polyhydroxy bicarboxyliques et d'acides polyhydroxy tricarboxyliques.
Particulièrement parmi les α-hydroxyacides ou leurs esters on peut citer : les acides glycolique, dioïque comme l'acide octadécène dioïque ou Arlatone dioc DCA vendu par la société Uniqema, citrique, lactique, tartrique, malique ou mandélique, leur esters comme le tartrate de dialkyle (C12/C13) ou Cosmacol ETI, le citrate de tri-alcools C12-13 ramifiés ou Cosmacol ECI commercialisé par la société SASOL.
Parmi les β-hydroxyacides on peut citer : l'acide salicylique et ses dérivés (dont l'acide n-octanoyl 5-salicylique).
Parmi les α-cétoacides on peut citer l'acide ascorbique et ses dérivés.
Parmi les autres agents desquamants on peut citer : les acides pyruvique, gluconique, glucuronique, oxalique, malonique, succinique, acétique, gentisique, cinnamique, azélaique ; le phénol ; la résorcine ; l'urée et ses dérivés, l'hydroxyéthyl urée ou hydrovance® de chez NATIONAL STARCH ; les oligofucoses ; l'acide jasmonique et ses dérivés ; l'acide ascorbique et ses dérivés, l'acide trichloracétique ; l'extrait de Saphora japonica et le résvératrol.
Parmi les agents desquamants, ceux capables d'agir sur les enzymes impliqués dans la desquamation ou la dégradation des cornéodesmosomes peuvent également être susceptibles de provoquer une irritation de la peau.
Parmi ceux-ci, on peut notamment citer les agents chélatants des sels minéraux tels l'EDTA; l'acide N-acyl-N,N',N'éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP 0 852 949, ainsi que le méthyl glycine diacétate de sodium commercialisé par BASF sous la dénomination commerciale TRILON M^{®}); le miel ; les dérivés de sucre tels que l'O-octanoyl-6-D-maltose, le O-linoleyl-6-D-glucose et la N-acétyl glucosamine.
Les rétinoïdes sont également des composés susceptibles de provoquer une irritation de la peau. On peut par exemple citer parmi eux le rétinol et ses esters, le rétinal, l'acide rétinoïque et ses dérivés tels que ceux décrits dans les documents FR-A-2 570 377, EP-A-199 636, EP-A-325 540, EP-A-402 072, et l'adapalène.

Les sels et dérivés, comme les formes cis ou trans, les mélanges racémiques, les formes dextrogyres ou levogyres des composés cités précédemment sont aussi considérés comme des composés susceptibles de provoquer une irritation de la peau.

D'autres actifs dermatologiques ou cosmétiques susceptibles de provoquer une irritation de la peau sont également cités ci-après :
- l'urée et ces dérivés comme l'hydroxyéthyl urée ou hydrovance® de NATIONAL STARCH,
- certaines vitamines telles que la vitamine D et ses dérivés tels que la vitamine D3, la vitamine D2, le calcitriol, le calcipotriol, le tacalcitol, la 24,25-diOH vitamine D3, la 1-OH vitamine D2 et la 1,24-diOH vitamine D2 ; la vitamine B9 et ses dérivés,
- les peroxydes comme le peroxyde de benzoyle, l'eau oxygénée,
- les antichutes tels que le minoxidil et ses derivés tel que l'aminexyl,
- les teintures et les colorants capillaires comme les aminophénols et leurs dérivés tels que la para-phénylène diamine (p-PDA), la N-phenyl p-PDA, le toluène 2,5-diamine sulfate, la méta-phénylène diamine (m-PDA), la toluène 3,4-diamine et l'ortho-phénylène diamine (o-PDA),
- les agents anti-transpirants comme les sels d'Aluminium, tel que l'hydroxychlorure d'aluminium,
- les déodorants,
- les actifs dépilatoires et/ou de permanentes tels que les thioglycolates, l'ammoniaque,
- le thioglycolate et ses sels,
- le phénoxyéthanol,
- le 1,2-pentanediol,
- les solutions alcooliques parfumantes (parfums, eaux de toilette, après rasage, déodorants)
- les anthralines (dioxyanthranol),
- les anthranoïdes (par exemple ceux décrits dans le document EP-A- 319028),
- les sels de lithium,
- les dépigmentants (ex : hydroquinone, vitamine C à forte concentration, acide kojique),
- certains actifs amincissants à effet chauffant,
- les nicotinates et leurs dérivés,
- la capsaïcine,
- les actifs antipoux (pyréthrine),
- les antiprolifératifs tels que le 5-fluoro uracile ou le méthotrexate,
- les agents antiviraux,
- les antiparasitaires,
- les antifongiques,
- les antiprurigineux,
- les antiséborrhéiques,
- certaines filtres solaires,
- les propigmentants tels que les psoralènes et les méthylangécilines, et
- leurs mélanges.

Comme conservateurs, on peut citer le phénoxyéthanol, la chorehexidine et le chlorure de benzalkonium.

Comme tensioactifs, on peut citer les tensioactifs anioniques, cationiques et amphotères, plus particulièrement les tensioactifs anioniques tels que les alkyl sulfates et alkyl éther sulfates comme le lauryl sulfate et le lauryl éther sulfate, et leurs sels notamment de sodium.

Selon un mode de réalisation préféré de l'invention, le composé susceptible de provoquer une irritation de la peau est choisi parmi les rétinoïdes, les α-hydroxyacides, les β-hydroxyacides, les acides dicarboxyliques saturés et insaturés tels que l'acide octadécène dioique ou Arlatone DIOC DCA vendu par la société Uniqema, les tensioactifs anioniques, cationiques ou amphotères, l'acide n-octanoyl 5-salicylique, les actifs antiperspirants tels que les sels d'aluminium, l'acide (N-2 hydroxyéthylpiperazine-N-2-éthane) sulfonique (HEPES) et l'acide cinnamique.

Le composé susceptible de provoquer une irritation de la peau peut être présent dans la composition selon la présente invention dans une quantité suffisante pour provoquer une réaction d'irritation de la peau. A titre d'exemple, il peut être présent dans une teneur allant de 0,0001 à 70% en poids, de préférence de 0,01 à 50% en poids et mieux de 0,1 à 30% en poids par rapport au poids total de la composition.

Selon un autre de ses objets, la présente invention se rapporte à un procédé cosmétique de traitement des peaux et/ou muqueuses et/ou semi-muqueuses et/ou cuir chevelus sensibles qui peut être mis en oeuvre notamment en appliquant les compositions telles que définies ci-dessus, selon la technique d'utilisation habituelle de ces compositions.

Par exemple : par application, c'est à dire par étalement sur une zone du corps, voire par massage pour faire pénétrer, de crèmes, de gels, de sérums, de lotions, de laits de démaquillage ou de compositions après-solaires sur la peau ou sur les cheveux secs, application d'une lotion pour cheveux sur cheveux mouillés, de shampooings, ou encore application de dentifrice sur les gencives.

Le procédé selon l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Ce procédé est avantageusement mis en oeuvre avant et/ou après une étape de peeling mécanique ou chimique, d'exfoliation de la peau. En effet, par ses vertus apaisantes, l'extrait bactérien selon l'invention prévient, soulage ou traite toute réaction d'inconfort comme les sensations dysesthésiques ou les signes d'irritation...

Selon une variante de l'invention, le procédé comprend en outre une étape de peeling ou d'abrasion de la peau.

L'invention se rapporte également à un extrait de bactérie filamenteuse n on photosynthétique susceptible d'être obtenu par le procédé comprenant au moins une étape de culture sur un milieu contenant une eau thermale non sulfureuse tel que décrit dans ce qui précède, pour son utilisation comme agent de traitement et/ou de prévention des signes associés à une peau sensible, et/ou comme agent apaisant et/ou pour diminuer ou prévenir les signes de l'inflammation.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés, avant leur mise en forme, dans l'ordre et dans des conditions facilement déterminées par l'homme de l'art.
Les exemples ci-après sont présentés à titre illustratif et non limitatif du domaine de l'invention.

### Exemple 1 - Préparation d'un extrait bactérien selon l'invention : biomasse de Vitreoscilla filiformis cultivée sur un milieu enrichi en eau thermale de La Roche Posay

Préparation du milieu de culture :
Composition :
* Extrait de levure 2 à 3 g
* Peptone Papaïnique de soja 2 à 3 g
* Glucose 2 à 3 g
* Micro élément de Heller 2 ml
* CaCl₂, 2H2O 66.21 mg
* Eau thermale La Roche Posay 13-14 ml.
Cette solution mère sera diluée par de l'eau osmosée dans un rapport de 1/75 avant stérilisation.

Le pH du milieu est ajusté à 5,00 par d'une solution molaire H₃PO₄. Le milieu est stérilisé par autoclavage à 121°C pendant 30 minutes. Après refroidissement à température ambiante, le pH est réajusté à 7,20 par rajout d'une solution molaire de KOH.

### Culture :

Après ensemencement à 1% du milieu avec la souche *Vitreoscilla filiformis* déposée à l'ATCC sous le n°15551, la culture est mise en agitation orbitale à 100 tours / min et à 26°C. Après 48 heures de croissance, la culture est centrifugée à 8000 g pendant 15 minutes. Les culots sont récupérés puis autoclavés à 121°C pendant 30 minutes. Cette biomasse peut être utilisée pour des tests d'évaluation.

### Exemple 2 - activité de l'extrait bactérien selon l'invention (biomasse de Vitreoscilla filiformis cultivée sur un milieu enrichi en eau thermale de La Roche Posay)

La capacité d'une telle biomasse cultivée dans une eau thermale (eau de La Roche Posay) à moduler l'inflammation neurogène a été réalisée.

### Résultats expérimentaux pour quatre produits évalués :

### Les produits évalués sont les suivants

**D8 *:*** biomasse de *Vitreoscilla filiformis* cultivée sur un milieu supplémenté en Sr, Se et Zn
**B51** *:* biomasse de *Vitreoscilla filiformis* cultivée sur un milieu enrichi en eau thermale de La Roche Posay.
**A28** *:* biomasse de *Vitreoscilla filiformis* cultivee sur un milieu H₂0 osmosé
**E1** *:* biomasse de *Vitreoscilla filiformis* cultivee sur un milieu supplémenté en Sr.

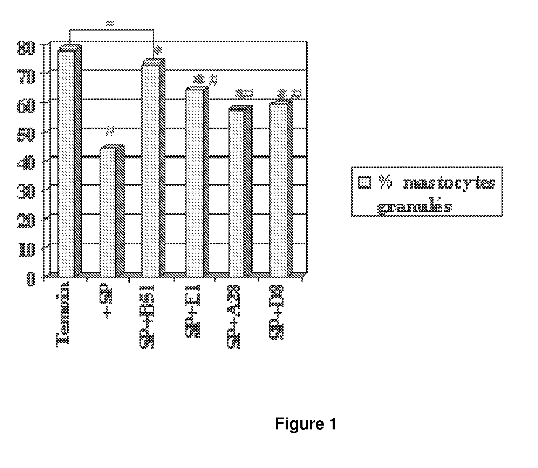

La **figure 1** montre que les 4 préparations sont statistiquement différentes par rapport à la peau traitée par la Substance P
La fraction B51 est la seule qui restaure une fonction normale.
** p<0, 05 versus peau traité* / ***dièse** p<0, 05 versus peau traitée à la substance P*

On observe ainsi que le traitement d'un explant de peau humaine en survie avec une biomasse de *Vitreoscilla filiformis* cultivée sur un milieu enrichi en eau thermale de La Roche Posay protège complètement le tissu d'une degranulation mastocytaire et du re-largage d'histamine qui fait suite à un stress neurogène induit par la substance P.
*A contrario*, la biomasse cultivée sur le même milieu de culture obtenu avec de l'eau distillée uniquement présente une activité sur ce process neurogène moins importante.
Ainsi, la biomasse de *Vitreoscilla filiformis* cultivée sur un milieu enrichi en eau thermale de La Roche Posay, présente une activité protectrice statistiquement supérieure à tel point que la peau stressée artificiellement par la substance P ne se distingue plus d'une peau non stressée en présence de l'extrait bactérien selon l'invention.

### Exemple 3 - Compositions

| **Lotion pour le visage des peaux sensibles :** | |
|---|---|
| Extrait selon l'exemple 1 | 5,00 |
| Gluconate de magnésium | 3,00 |
| Lactate de calcium | 2,00 |
| Antioxydant | 0,05 |
| Isopropanol | 40,0 |
| Conservateur | 0,30 |
| Eau | qsp 100 % |

| **gel pour le soin du visage des peaux sensibles :** | |
|---|---|
| Nitrate de strontium | 4,00 |
| Poudre d'une biomasse de *Vitreoscilla filiformis* cultivée sur un milieu enrichi en eau thermale de La Roche Posay | 5,00 |
| Hydroxypropylcellulose (Klucel H^{®} vendu par la société HERCULES) | 1,00 |
| Vitamine E | 2,50 |
| Antioxydant | 0,05 |
| Isopropanol | 40,00 |
| Conservateur | 0,30 |
| Eau | qsp 100 % |

## Revendications

1. Utilisation d'au moins un extrait de bactérie filamenteuse non photosynthétique et non fructifiante cultivée sur une eau minérale et/ou thermale non sulfureuse comme agent pour prévenir et/ou traiter les peaux et/ou cuirs chevelus sensibles.

2. Utilisation selon la revendication 1, **caractérisée en ce que** ladite bactérie est *Vitreoscilla filiformis*.

3. Utilisation selon la revendication 1 ou 2, **caractérisée en ce que** ladite eau est l'eau de La Roche Posay.

4. Utilisation selon l'une quelconque des revendications précédentes, **caractérisée en ce que** l'extrait est présent à raison de 0,001 à 20% en poids par rapport au poids total de la composition.

5. Utilisation selon l'une quelconque des revendications précédentes, comme agent pour prévenir et/ou traiter les peaux intolérantes ou irritables.

6. Utilisation selon l'une quelconque des revendications précédentes comme agent pour traiter les sensations dysesthésiques des peaux sensibles.

7. Utilisation selon la revendication 6, pour traiter les sensations choisies parmi les picotements et/ou les fourmillements et/ou les démangeaisons et/ou les échauffements et/ou l'inconfort cutané et/ou les tiraillements de la peau.

8. Utilisation d'au moins un extrait de bactérie filamenteuse non photosynthétique et non fructifiante cultivée sur une eau minérale et/ou thermale non sulfureuse comme agent apaisant.

9. Utilisation selon l'une quelconque des revendications précédentes, **caractérisée en ce que** ledit extrait est associé à au moins un composé choisi parmi les vitamines B3, B5, B6, B8, C, ou PP, la niacine, les protéines ou les hydrolysats de protéine, les acides aminés, les polyols, l'urée, l'allantoïne, les sucres et les dérivés de sucre, les vitamines hydrosolubles, l'amidon, des extraits bactériens ou végétaux, le rétinol (vitamine A) et ses dérivés, le tocophérol (vitamine E) et ses dérivés, les céramides, les huiles essentielles et des agents actifs destinés notamment à la prévention et/ou au traitement des affections cutanées.

10. Composition comprenant au moins un extrait de bactérie non photosynthétique et non fructifiante cultivée sur une eau minérale et/ou thermale non sulfureuse en association avec au moins un autre inhibiteur de neuropeptides et/ou un ingrédient susceptible de provoquer une irritation.

11. Composition selon la revendication 10, **caractérisée en ce que** ladite bactérie est *Vitreoscilla filiformis.*

12. Composition selon la revendication 10 ou 11, **caractérisée en ce que** ladite eau est l'eau de La Roche Posay.

13. Composition selon l'une quelconque des revendications 10 à 12, **caractérisée en ce que** l'extrait est présent à raison de 0,001 à 20% en poids par rapport au poids total de la composition.

14. Procédé cosmétique de traitement des peaux et/ou muqueuses et/ou semi-muqueuses et/ou cuir chevelus sensibles comprenant au moins une étape d'application d'au moins un extrait de bactérie non photosynthétique et non fructifiante cultivée sur une eau minérale et/ou thermale non sulfureuse.

15. Procédé selon la revendication 14, **caractérisé en ce que** ladite bactérie est *Vitreoscilla filiformis.*

16. Procédé selon la revendication 14 ou 15, **caractérisée en ce que** ladite eau est l'eau de La Roche Posay.

17. Procédé selon l'une quelconque des revendications 14 à 16, **caractérisée en ce que** l'extrait est présent à raison de 0,001 à 20% en poids par rapport au poids total de la composition.
